Europäisches Patentamt

European Patent Office

Office européen des brevets

(11) Publication number: **0 000 847**
**B1**

# EUROPEAN PATENT SPECIFICATION

(45) Date of publication of patent specification: **16.09.81**

(51) Int. Cl.³: **C 07 C 21/06, C 07 C 17/34**

(21) Application number: **78300280.1**

(22) Date of filing: **14.08.78**

(54) Vinyl chloride monomer production.

(30) Priority: **16.08.77 GB 3427077**

(43) Date of publication of application:
**21.02.79 Bulletin 79/4**

(45) Publication of the grant of the European patent:
**16.09.81 Bulletin 81/37**

(84) Designated Contracting States:
**BE DE FR NL SE**

(56) References cited:
**DE - B - 1 075 592**
**FR - A - 1 306 945**
**GB - A - 1 168 329**
**GB - A - 1 265 245**
**US - A - 2 569 923**

**ULLMANS ENCYKLOPAEDIE DER TECHNISCHEN CHEMIE, 3. Auflage, Band 18, 1967, Urban & Schwarzenberg, München-Berlin-Wien, pages 91—93**

(73) Proprietor: **BP Chemicals Limited**
**Britannic House Moor Lane**
**London, EC2Y 9BU (GB)**

(72) Inventor: **Burch, Derek Arthur**
**BP Research Centre Chertsey Road**
**Sunbury-on-Thames Middlesex, TW16 7LN (GB)**
Inventor: **Butler, Edward John**
**BP Research Centre Chertsey Road**
**Sunbury-on-Thames Middlesex, TW16 7LN (GB)**
Inventor: **Capp, Clifford William**
**BP Research Centre Chertsey Road**
**Sunbury-on-Thames Middlesex, TW16 7LN (GB)**

(74) Representative: **Harry, John et al,**
**BP INTERNATIONAL LIMITED Patents and Licensing Division Chertsey Road**
**Sunbury-on-Thames Middlesex TW16 7LN (GB)**

Courier Press, Leamington Spa, England.

## Vinyl chloride monomer production

The present invention relates to an essentially non-catalytic process for the preparation of vinyl chloride by the dehydrochlorination of mixtures of isomeric dichloroethanes.

It is known that vinyl chloride can be produced by the pyrolytic dehydrochlorination of 1,2-dichloroethane. The process involves the passing of 1,2-dichloroethane through a small diameter reaction tube in which it is heated to elevated temperatures to bring about the dehydrochlorination thereof. In contrast to this, it has not hitherto been possible to convert the isomeric 1,1-dichloroethane, which is a byproduct of the pyrolysis of 1,2-dichloroethane, into vinyl chloride in economic yields. US—A—2569923 describes a method of pyrolysing a mixture of 90% 1,1-dichloroethane and 10% 1,2-dichloroethane. However this mixture results only in a 35% conversion.

It has now been found that mixtures of these isomers can be pyrolysed without impairing the quality or yield of vinyl chloride monomer produced by regulating the concentration of the two isomers in the mixture being pyrolysed.

Accordingly, the present invention is a process for producing vinyl chloride comprising pyrolysing at elevated temperature 1,2-dichloroethane mixed with between 0.1 and 10% by weight of 1,1-dichloroethane.

The above reaction may be carried out in conventional tubular reactors as normally employed for the pyrolysis of 1,2-dichloroethane. The pyrolysis of mixtures of 1,1-dichloroethane and 1,2-dichloroethane in the specified range of concentrations significantly increases the conversion of 1,1-dichloroethane from about 20% (for the pure isomer) to above 90% (when present in the isomeric mixtures). Both below and above this range the conversion of the 1,2-isomer is depressed relatively. The amount of the 1,1-dichloroethane in the isomeric mixture being pyrolysed is preferably between 1 and 8%. The 1,1-isomer may be mixed with the pyrolysis feed prior to the introduction thereof into the reaction zone. Alternatively it may be incorporated in the pyrolysis feed in the reaction zone itself at the time of pyrolysis.

The pyrolysis of the isomeric mixtures may be carried out in packed or unpacked tubular reactors. Where packed reactors are used they may suitably be packed with ceramic spheres.

The pyrolysis of the isomeric mixture of dichloroethanes according to the present invention is suitably carried out at a temperature of between 450° and 550°C, preferably between 500° and 535°C.

The pyrolysis of the isomeric mixture of dichloroethanes may be carried out at subatmospheric, atmospheric or super-atmospheric pressures. It is, however, preferred to use super-atmospheric pressure, preferably below 35 atmospheres.

The pyrolysis of the isomeric mixture of dichloroethanes may be carried out in a continuous manner.

The advantages of the present invention are illustrated with reference to the following Examples.

### EXAMPLES

The apparatus, run at atmospheric pressure, consisted of feed reservoir, metering pump, vaporiser, cracker tube, coolers, organic product receiver, hydrogen chloride scrubbing column, scrub liquor reservoir and wet gas meter. There was also a nitrogen diluent/carrier feed applied to the vaporiser inlet.

Feedstocks comprising pure 1,2-dichloroethane (1,2-EDC), 1,2-dichloroethane with up to 10 per cent by weight of 1,1-dichloroethane (1,1-DCE) and also pure (98.2% by weight) 1,1-DCE were pyrolysed at a peak temperature of 500°C and a contact time of 4.5 seconds under conditions found to give a 50 per cent by weight conversion of pure 1,2-dichloroethane.

Analysis of feedstock, organic condensate, scrub liquor and vent gas were used to calculate the conversions tabulated below.

| 1,1–DCE (weight %) | Conversions (weight %) | | | | | Balances (weight %) | |
|---|---|---|---|---|---|---|---|
| | 1,2–EDC | 1,1–DCE | 1,2–EDC +1,1–DCE | to HCl | to VCM | Mass | Carbon |
| Zero | 52.3 | – | – | 52.5 | 50.5 | 100 | 99.6 |
| 1.5 | 56.0 | 98.6 | 56.8 | 56.8 | 55.0 | 100 | 99.2 |
| 3.9 | 52.3 | 99.4 | 54.2 | 53.0 | 55.4 | 100 | 100 |
| 5.9 | 48.5 | 99.6 | 51.5 | 50.5 | 50.9 | 100 | 100 |
| 7.9 | 50.9 | 99.7 | 54.8 | 54.0 | 54.3 | 100 | 100 |
| 9.9 | 47.1 | 99.8 | 52.3 | 51.5 | 51.8 | 100 | 100 |
| 98.2 | – | 19.3 | – | 20.0 | 19.1 | 100 | 99.8 |

## Claims

1. An essentially non-catalytic process for producing vinyl chloride by pyrolysing 1,2-dichloroethane at elevated temperature characterised in that 1,2-dichloroethane is mixed with between 0.1 and 10% by weight of 1,1-dichloroethane.

2. A process according to claim 1 characterised in that the amount of 1,1-dichloroethane in the isomeric mixture being pyrolysed is between 1 and 8% by weight.

3. A process according to claim 1 or claim 2 characterised in that the pyrolysis of the isomeric mixture of dichloroethanes is carried out at a temperature between 450°C and 550°C.

4. A process according to claim 3 characterised in that the pyrolysis of the isomeric mixture of dichloroethanes is carried out at a temperature between 500 and 535°C.

5. A process according to any of the preceding claims characterised in that the pyrolysis of the isomeric mixture of dichloroethanes is carried out at superatmospheric pressures.

6. A process according to claim 5 characterised in that the superatmospheric pressure is below 35 atmospheres.

7. A process according to any of the preceding claims characterised in that the pyrolysis of the isomeric mixture of dichloroethanes is carried out in a continuous manner.

## Revendications

1. Procédé essentiellement non catalytique pour produire du chlorure de vinyle par la pyrolyse du 1,2-dichloréthane à température élevee, caractérisé en ce qu'au 1,2-dichloréthane est mélangé de 0,1 à 10% en poids de 1,1-dichloréthane.

2. Procédé selon la revendication 1, caractérisé en ce que la proportion du 1,1-dichloréthane dans le mélange des isomères soumis à la pyrolyse se situe entre 1 et 8% en poids.

3. Procédé selon l'une des revendications 1 et 2, caractérisé en ce que la pyrolyse du mélange des dichloréthanes isomères est effectuée à une température comprise entre 450°C et 550°C.

4. Procédé selon la revendication 3, caractérisé en ce que la pyrolyse du mélange des dichloréthanes isomères est effectuée à une température comprise entre 500° et 535°C.

5. Procédé selon l'une quelconque des revendications précédentes, caractérisé en ce que la pyrolyse du mélange des dichloréthanes isomères est effectuée à des pressions supérieures à la pression atmosphérique.

6. Procédé selon la revendication 5, caractérisé en ce que la pression supérieure à la pression atmosphérique est inférieure à 35 atmosphères.

7. Procédé selon l'une quelconque des revendications précédentes, caractérisé en ce que la pyrolyse du mélange des dichloréthanes isomères est effectuée de façon continue.

## Patentansprüche

1. Ein im wesentlichen nicht-katalytisches Verfahren zur Herstellung von Vinylchlorid durch Pyrolyse von 1,2-Dichloräthan bei erhöhter Temperatur, dadurch gekennzeichnet, daß 1,2-Dichloräthan mit zwischen 0,1 und 10 Gew.-% 1,1-Dichloräthan gemischt wird.

2. Ein Verfahren gemäß Anspruch 1, dadurch gekennzeichnet, daß die Menge von 1,1-Dichloräthan in der zu pyrolysierenden Iso-

merenmischung zwischen 1 und 8 Gew.-% liegt.

3. Ein Verfahren gemäß Anspruch 1 oder 2, dadurch gekennzeichnet, daß die Pyrolyse der Isomerenmischung der Dichloräthane bei einer Temperatur zwischen 450° und 550°C durchgeführt wird.

4. Ein Verfahren gemäß Anspruch 3, dadurch gekennzeichnet, daß die Pyrolyse der Isomerenmischung der Dichloräthane bei einer Temperatur zwischen 500° und 535°C durchgeführt wird.

5. Ein Verfahren gemäß einem der vorgehenden Ansprüche, dadurch gekennzeichnet, daß die Pyrolyse der Isomerenmischung der Dichloräthane bei überatmosphärischen Drucken durchgeführt wird.

6. Ein Verfahren gemäß Anspruch 5 dadurch gekennzeichnet, daß der überatmosphärische Druck unterhalb 35 Atmosphären liegt.

7. Ein Verfahren gemäß einem der vorgehenden Ansprüche dadurch gekennzeichnet, daß die Pyrolyse der Isomerenmischung der Dichloräthane kontinuierlich durchgeführt wird.